# EUROPEAN PATENT APPLICATION

(11) **EP 2 465 469 A1**
(43) Date of publication of application: **20.06.2012**
(21) Application number: 10195832.0
(22) Date of filing: 19.12.2010
(51) Int. Cl.: A61D 17/00, G01N 33/558

(54) **Device for predicting the optimum insemination time for an animal**

(71) Applicant: Vankrieken, Leo, 3920 Lommel (BE); Collee, Marleen Germaine J., 1080 Brussel (BE); Warmoes, Pierre, 7063 Soignies (BE)
(72) Inventor: Vankrieken, Leo, 3920, Lommel (BE); Collee, Marleen Germaine J., 1080, Brussel (BE); Warmoes, Pierre, 7063, Soignies (BE)
(74) Representative: Callewaert, Raf

(57) **Abstract**

Method and device for predicting optimum insemination time of an animal and/or pregnancy detection, by determining in a body fluid sample of said animal the presence and/or concentration of at least one analyte of interest selected from the group comprising progesterone, lutenizing hormone LH, follicle stimulating hormone FSH, prolactin PRL, sex hormone binding globuline SHBG, estradiol and/or testosterone, wherein a lateral flow immunoassay is used.

## Description

The present invention relates to the detection of ovulation and corresponding insemination time problems in animals, particularly in cows, pigs, horses and dogs, and provides methods and technologies by which the optimal times of fertilization detection and pregnancy detection can be maximized in these different mammalian animal species by performing the test near to the animal.

Reproductive efficiency in breeding is very crucial to the economic success of the breeding society. One of the most important reproductive technologies in the breeding industry is Artificial Insemination (AI).

Artificial insemination in veterinary species has become a routine common method used to breed cattle, horses, pigs and dogs. AI reduces the incidence of sexually transmitted diseases and the process involves taken semen collected from the animal and depositing it in the female animal. The primary advantage of AI is that it allows breeders to select from a larger population of the respective breeding males and making superior genetics available worldwide. In general the following advantages are obtained by AI:
- to improve the genetic quality;
- to increase consistency and vale of the newborns;
- to easier access to superior and proven male animals at an affordable price;
- to eliminate the transport and housing of the male animal at the female's site;
- to reduce labour and costs associated;
- to program in a much better way the breeding and birth season;
- to avoid the housing and expenses associated with the presence of the male animal.

However, properly executing an AI program requires specialized knowledge because different steps are involved. The success of AI rated by the pregnancy outcome depends mainly on 4 important steps:
- the inherent fertility grade of the sperm;
- the proper handling of the sperm prior to insemination;
- Insemination at the right time during the estrus cycle or after ovulation (species dependent);
- Proper semen deposition.

In general, it is well understood that a positive fertilization outcome will be achieved by having fertile sperms present in the oviduct when ovulation occurs. The life time of the oocyte is relatively short and therefore sperm cells should be present near to the follicle or should reach sufficiently ahead of ovulation to effect proper fertilization. In the routine practice, ovulation time and the correct AI are very difficult to predict routinely in a herd of hundreds of cows or sows. As an example: usually cows come in estrus after every 21 days if not pregnant. Estrous behavior is used to determine when a cow or a sow should be examinated. Farmers will observe the behaviour of the cow in order to detect the period of heat. The estrus in cows is detected in a very subjective way: the female will stand to be mounted by another animal. Cows first seen in the estrus in the morning should be inseminated in the afternoon of the same day; those first observed in estrus in the afternoon should be inseminated next morning. Mostly AI is repeated in the same animal.

With natural breeding programs, the fertility rate is about 50 to 60% in cows and can be increased with proper programming up to 70-85%.

Senger published in 1994 (The estrus detection problem: new concepts, technologies, and possibilities. J Dairy Sci, 1994:77:3185) that the US dairy industry loses more than 300 Million US$ annually due to failure and/or misdiagnosis of the estrus period.

In general, looking to the cycles in animals it is well understood that ovulation is initiated by a surge of luteinizing hormone (LH) from the pituitary gland in the brain. This surge, reflected by elevated LH levels, results in the rupture of the follicle and the release of the ovum/oocyte from the ovary. Ovulations occur approximately 28 to 32 hours after the onset of estrus in dairy cows (Trimberger: Breeding efficiency in dairy cattle from artificial insemination at various intervals before and after ovulation. 1948; Univ Nebraska Ag. Exp. Sta. Res. Bull. 153:3 & Walker et al.: Time of ovulation relative to mounting activity in the dairy cattle. 1996 J. Dairy Sci.:79:155). After ovulation, there is only a short period when the ova can be fertilized: between 6 and 12 hours after ovulation (Brackett et al. Fertilization and early development of cow ova. 1980: Biol. Reprod.:23:189).

For more than 50 years, researchers have investigated the optimal time at which to inseminate cows relative to the stage of ovulation or to the stage of the estrus. Different subjective methods, technologies and observations times of the cows were published and are used today in routine practice. Many farmers are observing their cows for symptoms of 'heat':
- the animal will be in excited condition (restless and nervousness);
- the animal will reduce the intake of feed;
- a peculiar movement of limbo sacral region will be observed;
- animals in heat will be licking and smelling other animals;
- the animal will stand still when another animal try to mount;
- frequent urination;
- clear mucous discharge from the vulva;
- swelling of the vulva;
- tail will be raised;
- milk production in cows will be decreased;
- etc ...

Controlled investigations are mandatory to determine the time of AI. Many studies revealed that conception rate is lower when cows are bred prior mid estrus or later than 6 hours after cessation of the estrus (standing heat in this case). Maximal conception is obtained when cows are inseminated between mid estrus and the end of the standing estrus, with good results up to 6 hours after estrus.

In order to find a more objective way of determining the Heat Time, several commercial products are available that measure the mounting activity or other body activities (restless, body temperature, heart rate changes, ...) by placing electronic measurement devices on the animal. Other published studies describe the measurement of electric resistance of the vaginal mucus (W. Leidl et al, Theriogeology 1976) or the use of biosensors (E.H. Gils et al., J Immunology Methods 2002) for progesterone measurements in milk for heat detection and pregnancy prediction.

Sometimes and very rarely, the use of animal specific assays such as LH or more generic, non-animal specific tests, such as Progesterone or Estradiol measurements are carried out by medical or veterinarian laboratories on a serum or plasma sample of the animal. This means that different steps are involved:
- a blood sample needs to be collected by the veterinarian doctor;
- the sample needs to be transferred to the laboratory;
- the blood sample needs to be centrifuged;
- a specific hormonal measurement needs to be carried out:
   Concerns:
      - Labs don't perform such a test every day of the week;
      - often labs are not working during the weekend, but animals are ovulating also during the weekend;
      - the time difference between the moment of the sample withdrawal and the arrival at the lab can take several hours, sometimes more than 12 hour.

Based on all these unpractical laboratory settings and expensive costs (hormonal measurements and transportation costs) and also taken into account the unpractical use in sow-farms (pigs) no use of these technologies are seen in the real, routine practical settings of farms and breeding stations for porcine, bovine, horses and rarely done in canine species.

Previous development of rapid tests by using test strips failed due to e.g. a lack of sensitivity, specificity and the need for relatively large samples of material taken from the animal e.g. several test tubes of whole blood.

The aim of the present invention is to offer a rapid test that allows:
- to measure the exact time of ovulation near to the animal;
- to determine the optimum time of artificial insemination in different veterinary species within a relative short time frame and near to the animal;
- to reduce the number of repetitive inseminations such as seen in sows, cattle, mares and bitches;
- to detect early onset of pregnancy;
- to use a minimum amount of whole blood, e.g. to use only a few drops;
- the test to be easily performed by at least: the farmer (e.g. in porcine and bovine industry), an inseminator (e.g. in bovine and equine industry), a veterinarian doctor (e.g. in bovine, equine and canine industry) or a horse breeder.

### FIGURES

Figure 1 shows the reproductive cycle of a cow.
Figure 2 shows the reproductive cycle of a sow.
Figure 3 shows the reproductive cycle of a mare.
Figure 4 show the use of a diagnostic kit with a lateral flow immunoassay device according to the invention.
Figure 5 shows a schematic representation of the interaction between an analyte of interest, i.e. progesterone, biotinylated progesterone tracer, biotin-specific molecule, and an analyte-specific molecule, i.e. anti progesterone antibody, in a competitive immunoassay.

A rapid quantitative or semi-quantitative detection of analytes, such as Pituitary hormones, LH, FSH, Prolactin, and/or Steroids, Progesterone, Estradiol, Total Estrogens and/or Testosterone and/or steroidal binding proteins SHBG (sex hormone Binding Globulin), each of these analytes separately or in combination, in blood or other biological fluids, allows for detection of ovulation, optimum insemination time and pregnancy in different mammalian veterinary species.

Progesterone, secreted by the corpus luteum, suppresses the further development of follicles and the secretion of estrogens. High levels of progesterone and low levels of estrogens prevent the animal from coming into heat. Progesterone is necessary for preparing the uterus to receive the fertilized egg and maintains the proper uterine environment for continuation of pregnancy. Progesterone has a quieting effect on the uterus so that there are no contractions which might disturb pregnancy. On the contrary, Estrogens cause contraction of the uterus near the time of estrus and ovulation, which aid in sperm transport.

However, the interpretation of Progesterone levels in dogs is different. For most bitches a progesterone level of 2.0 ng/ml indicates a surge of LH from the pituitary gland, which triggers the release of the eggs from the follicle. When progesterone is reaching 5 to 10 ng/ml, the bitch is ovulating. After ovulation, the released eggs continue to go through maturation until they are mature two or three days later. Once the maturation process is completed, the eggs are ready to be fertilized.

Therefore to accurately identify the time of ovulation, a series of progesterone measurements are conducted to map the progesterone levels

LH and Follicle Stimulating Hormone (FSH) are secreted in mammalians by the pituitary gland at the height of the brain. Each of these hormones is a two subunit glycoprotein of which the two subunits are non-covalently bound together: the so-called alpha and the beta subunits. The alpha subunits of both LH and FSH are nearly 100% identical; however the biological activity of the hormone resides in the beta subunit. LH and FSH vary between different species to some extent in its molecular size and amino-acid sequences and therefore are these hormones different between animal species and also different from human.

FSH is an important hormone that reflects follicular reserve on the ovaries and appropriate levels must be maintained between Day 7 and Day 10 for ovulation quality during that cycle. FSH stimulates the growth, development and functions of the follicle, while LH cause the follicle to rupture during ovulation and causes the subsequent development of the corpus luteum, with gradually increasing levels of Progesterone as a consequence.

High levels of Prolactin, a monomeric hormone, leads to infertility and animals can be treated with bromocryptine. Therefore Prolactin levels - if accurately measured - can be a useful tool to decide on treatment before any AI can be installed.

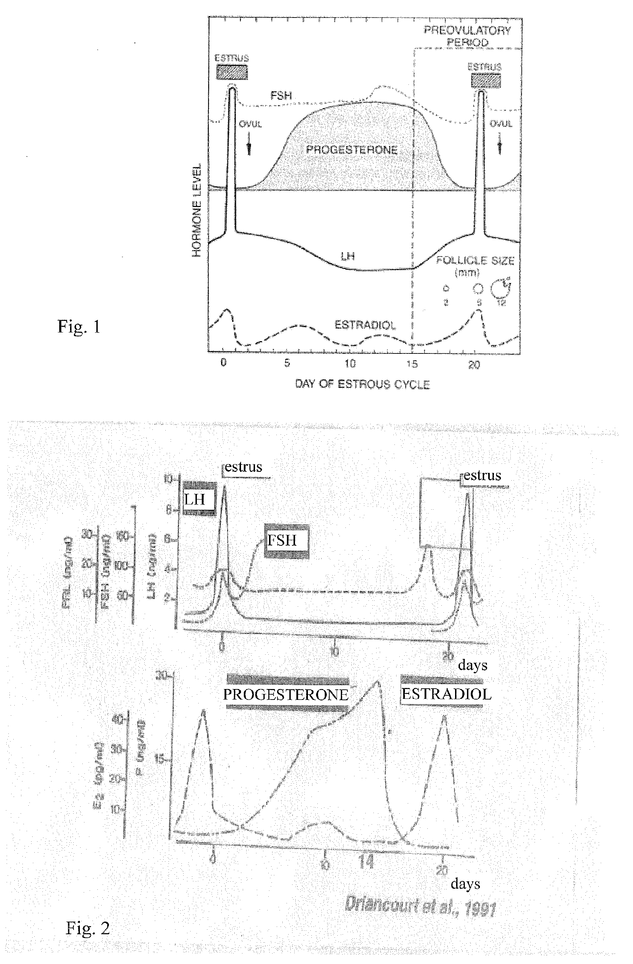

The reproductive cycle of a cow with an average cycle length of 21 days is illustrated in figure 1.

Days 0-1: The cow is in for estrus (standing heat) on Day 0 for an average of 18 hours (range 12 to 24 hours). About 12 hours after the end of the standing heat, the mature Graafian follicle ruptures (ovulates) in response to a surge of LH released by the pituitary gland.

Days 1 and 2: The cells that formerly lined the follicle change and become the lutein cells of the corpus luteum. This change in cell forms is caused by hormonal action, primarily that of LH.

Days 2 to 5: The corpus luteum grows rapidly in both size and function. Numerous follicles may be seen on the ovary at this stage, but by Day 5 they have begun to regress.

Days 5 to 16: The corpus luteum continues to develop and reaches its maximum growth and function about Day 10. It secrets the hormone progesterone, which inhibits (blocks) LH release by the pituitary gland. During this period, the ovaries are relatively inactive except for the functional corpus luteum. No follicles reach maturity and/or ovulate because of the existence of the high levels of progesterone.

Days 16 to 18: The corpus luteum regresses rapidly due to some luteolytic activity of the uterus. Evidence is increasing that this may be a prostaglandin.

Days 18 to 20: The corpus luteum is almost non-functional and this releases the blocking action of progesterone. Of the several follicles that commence growth, one becomes more prominent by a surge in rapid growth and activity. As the Graafian follicle grows, it secretes increasing amounts of estrogen. The remainder of the follicles regress.

Day 21 or 0: With the increase in estrogen release by the Graafian follicle and a corresponding decrease in progesterone by the regressing corpus luteum, estrus or heat will occur (cycle has now returned to Day 0). The high estrogen level in the blood triggers a release of LH near the end of heat. Following this surge in blood levels of LH, the mature follicle ruptures to release the egg and the cellular tissue left behind becomes luteinized in response to the stimulation of a hormonal complex to form a new corpus luteum (cycle has now returned to Days 1 and 2). Progesterone again becomes the dominant hormone.

It must be noted that the timing given for the preceding events is only approximate and differs for different cycle lengths.

The discussion of events occurring during the previous cycle was based on a full cycle in which pregnancy does not occur. If the egg is fertilized and begins development in the uterus, the corpus luteum does not regress but continues to function by secreting progesterone. No follicles develop to maturity and heat does not occur. Progesterone keeps the uterus quiet and thus provides the most favorable conditions for the developing fetus.

The hormonal changes during the cycle in a sow are represented in the diagram in figure 2.

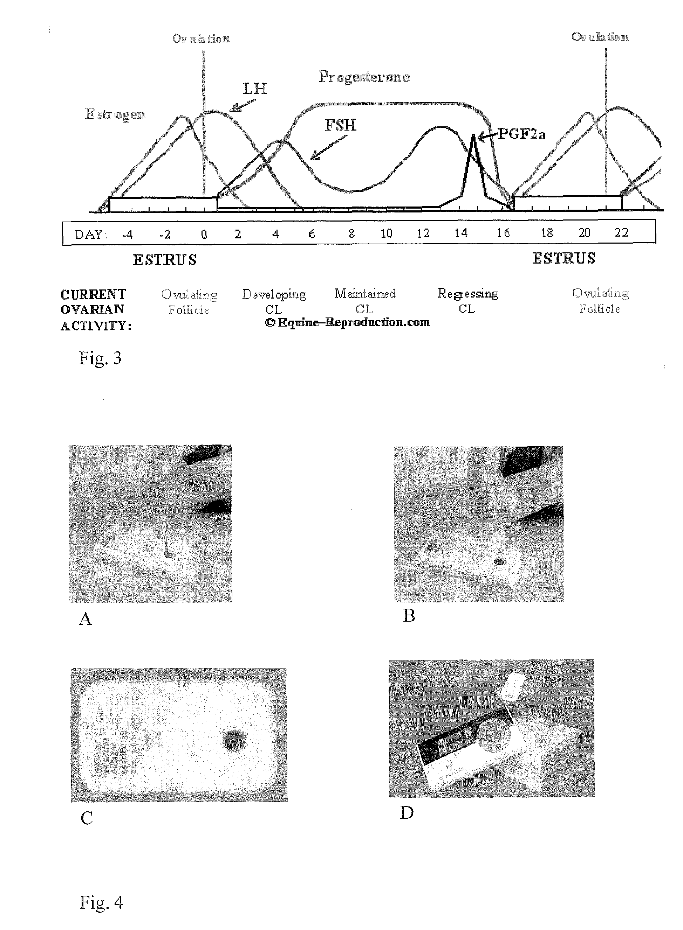

A schematic overview of the regular estrus cycle in mares is represented in figure 3.

Key features of these cycles are:
- The entire estrous cycle from ovulation to ovulation takes approximately 21 days;
- Ovulation of a follicle occurs, releasing the ova for potential fertilization;
- The cavity left behind on the ovary by the evacuated follicle fills rapidly with blood and in some cases forms a Corpus Hemorrhagicum prior to creation of luteal tissue to become a Corpus Luteum (CL) as a result of the presence of LH;
- By about 5 days after ovulation, the CL is fully functional and secreting progesterone. This is also known as the luteal phase;
- FSH action early in diestrus may produce a mid-cycle follicle that will sometimes ovulate, but more usually regresses;
- Around day 13 post-ovulation the endometrium of the uterus secretes PGF2α which causes the destruction ("lycing") of the CL, which will then permit the onset of estrus behavior;
- FSH activity in late diestrus will have caused the selection of a dominant follicle (or possibly two) which under the influence of increasing estrogen levels early in estrus will develop to reach ovulatory status approximately 21 days after the previous ovulation occurs.
- The mare will display receptive behavior (estrus) towards the stallion for 5-7 days and will ovulate in the last 24-48 hours of that display period.

It is thus an object of the present invention to obtain the highest rate of fertilization of an animal such as cow, mare, sow, and bitch by using a rapid test near to the animal to accurately predict in advance the time of ovulation by measuring LH and/or Estradiol or the time of artificial insemination by measuring Progesterone. Furthermore, the same rapid Progesterone test, near to the animal, can be used to confirm pregnancy in the animal.

The test according to the invention preferably uses whole blood without the need for plasma or serums such that blood sample do not need centrifugation. Alternative biological fluids such as for example urine, mucus or saliva, can be used also.

The test according to the invention allows using only a few drops of whole blood, without the need to take an entire classical blood tube from the animal. The drops of whole blood are brought immediately on the test unit such that the tests can be performed near to the animal.

The tests can be performed by a veterinarian doctor or by a farmer.

A specific rapid test according to the invention allows for measuring Progesterone near to the animal. The same assay can be used for different animal species.

Another specific rapid test according to the invention, detects LH near to the animal. The test may be animal specific, meaning the rapid LH test may be a different assay for e.g. cows, mares, sows and bitches.

Still another specific rapid test according to the invention, detects FSH near to the animal. The test may be animal specific, meaning the rapid FSH may be a different assay for e.g. cows, mares, sows and bitches.

Still another specific rapid test according to the invention, detects Estradiol near to the animal. The same assay that may be used between different animal species.

Still another specific rapid test according to the invention, detects Prolactin.

According to an aspect of the invention the rapid test is performed by using only a few drops of whole (total) blood directly applied to a lateral flow immunoassay wherein specific antibodies are used such as:
For Progesterone:
   - monoclonal antibodies anti-progesterone, or
   - polyclonal antibodies or
   - a combination (mixture) of monoclonal with polyclonal antibodies anti-progesterone

For LH:
- polyclonal antibodies (commercially available), however specific towards each of the animals.

For FSH:
- polyclonal antibodies (commercially available), however specific towards each of the animals.

For Estradiol:
- monoclonal antibodies anti-estradiol, or
- polyclonal antibodies or
- a combination (mixture) of monoclonal with polyclonal antibodies anti-estradiol

For Prolactine:
- polyclonal antibodies (commercially available)

For SHBG:
- polyclonal antibodies (commercially available)

According to another aspect of the invention the rapid test is performed by using only a few drops of whole (total) blood directly applied to a lateral flow immunoassay wherein a paper membrane is used for whole blood separation. Said membrane separates blood cells from blood fluid.

According to another aspect of the invention the rapid test is performed by the use of specific antibody coated paper membranes for antigen recognition.

According to another aspect of the invention the rapid test is performed in which after the incubation time, during which an Antigen-Antibody reaction takes place, and after the migration time, a colour will be developed. The intensity of the colour directly being correlated to concentration of the detected analyte, e.g. either progesterone, LH or Estradiol, depending on the used antibody.

According to another aspect of the invention the density of the colour developed during the test is read by a portable calibrated reader. Preferably, the OD (Optical Density) result from the reader is transformed into a semi quantitative level or a quantitative level.

According to another aspect of the invention the results are obtained with equivalent sensitivity and specificity as used by routine laboratories and results will be directly used for ovulation detection and/or determination of optimum time of insemination depending on the animal species.

More in detail, the present invention concerns rapid tests based on a so-called lateral flow immunoassay. Lateral flow immunoassays as such are widely known. Examples are described in e.g. US Patent No. 5,770,460, US Patent No. 6,656,744.

Lateral flow immunoassays - often called rapid tests - are simple one-step or two-step assays for the qualitative determination of analytes directly out of patient's samples. Quantitative interpretation of the results of rapid tests is also possible which is becoming more and more important in the field of point-of care (syn.: bedside) testing. Beside its fast performance the detection of analytes (small molecules like antibodies or other soluble analytes and antigens) directly from whole blood, urine or other body fluids without any further treatment is one of the major advantages of rapid tests.

In the present invention this approach is used in different veterinarian applications for different veterinarian species with respect to measurements of Progesterone, LH, FSH, Estradiol, Prolactin, Testosterone, SHBG and others near to the animal.

The rapid lateral flow test consists of a system of overlapping membranes containing the dried components needed for the test performance. These membranes are assembled to small strips which are placed into a plastic housing for better handling. This plastic housing with the membrane strips is a test unit.

The animal its sample material is loaded to a Sample Application Pad. This sample material may consist of a drop of blood. Figure 4A shows the transfer of a drop of blood to the test unit.

Further, preferably a liquid buffer is added (see figure 4B). This may consist of 2 drops of buffer with Biotinylated Tracer.

In the case of whole blood/capillary blood samples a separation of blood cells and plasma takes place. The liquid fraction of the animals sample diffuses through the so-called Conjugate Release Pad containing labelled detection molecules.

The conjugate molecules are specifically directed against the analyte of interest. The conjugate is re-dissolved and the analyte is specifically bound by the gold conjugate.

The analyted-gold conjugate complex further diffuses through the Analytical Membrane.

On this membrane mostly two lines are arranged one after the other:
1.- the Test Line containing analyte-specific molecules responsible for immobilizing the analyte-gold conjugate complexes;
2.- the Control Line fixing non-bound gold conjugate particles indicating the conjugate has overflown the Test Line.

The last component of the rapid test is the Wicking (or Sink) Pad, which simply collects the fluid running through the test system and prevents backflow of the fluid through the test system.

The result can be read from the test unit (see figure 4C). The colour intensity of the test line is directly proportional to the analyte concentration in the animals sample enabling (semi-)quantitative interpretation of the test result.

If the analyte of interest is available above the detection limit the Test Line and the Control Line are clearly visible; if the analyte is below the detection limit only the Control Line appears during test time.

Further the pads of the test unit may preferably be read in a calibrated Micro-Scan reader, as shown in figure 4D. Calibration is done for each of the specific hormones/steroids to be detected. The results are expressed in absolute values or in semi-quantitative results. Results are reported on the digital screen, or stored or printed.

The test results by using e.g. the calibrated reader may be based on colour density and may be presented as:
- semi-quantitative results (represented as a block-diagram and the number of blocks represent a concentration range)
- quantitative results in weight values (such as for example ng/ml, pg/ml,) or
- standard units (such as for example mU/ml or U/L or equivalent units).

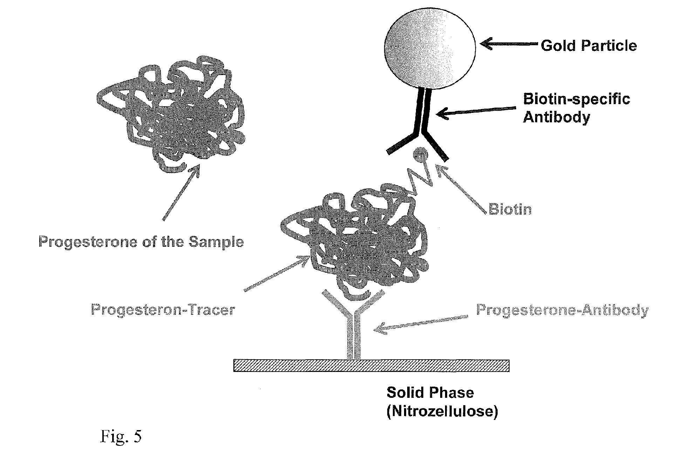

Figure 5 illustrates the working of a test for detection of Progesterone which uses a competitive immunoassay with golden particle.

The antigen in the blood sample, which is in this example Progesterone, will compete with the biotinylated antigen (or the labelled detection molecules / biotinylated Tracer) to bind them with the antibody coated on a solid layer of nitrocellulose. The conjugated molecules are specifically directed against the analyte of interest. This competitive binding occurs in the Conjugate Release Pad. After the required incubated time, the conjugate is redissolved and the analyte (the complex: antibody-labeled antigen) is at that moment specifically bound by the gold conjugated. This Progesterone-Gold conjugate complex further diffuses through the analytical membrane on which two lines are placed:
- the test line: on which the progesterone-specific molecules immobilize the progesterone-gold conjugate complexes
- the control line: on which non-bound conjugate particles are fixed indicating that the conjugate has overflown the test line and provides an indication that the test has been carried out.

The colour density (or intensity) of the test line is directly proportional to the progesterone concentration in the blood samples and provides a quantitative or semi-quantitative measurement if read in the calibrated reader.

Important in the rapid test are the specificities of the antibodies and the titers used. Antibodies that may be used in the tests according to the invention are as follows:
- Monoclonal anti-progesterone antibody with the following characteristics:

| **anti-Proqesterone** | Monoclonal Prog.11HS-BSA |
|---|---|
| Compound Cross-Reactivity % | X-1 |
| Progesterone | 100 |
| 17-Hydroxyprogesterone | 1 |
| 11-Hydroxyprogesterone | 25 |
| 5-alpha-pregnane-3,20 dionene | 10.5 |
| Corticosterone | 0.01 |
| Pregnenolone | 0.9 |
| Deoxycorticosterone | 0.3 |
| Deoxycortisol | 0.02 |
| Cortisol | |

- Polyclonal anti-progesterone antibody with the following characteristics:

| **anti-Proqesterone** | polyclonal sheep Prog.11 HS-KLH |
|---|---|
| Compound Cross-Reactivity % | P-1 |
| 20-α-Dihydroprogesterone | 0.03 |
| 20-β-Dihydroprogesterone | 3.27 |
| 5-α-Pregnan-3,20 dione | 3.51 |
| 17-α-Hydroxyprogesterone | 1.50 |
| Pregnonelone | 0.38 |
| Cortisol | 0.003 |
| 21-Deoxycortisol | 0.01 |
| 11-Deoxycortisol | 0.05 |
| Corticosterone | 0.30 |
| 11-Deoxycorticosterone | 0.83 |
| Androstenedione | 0.12 |
| Testosterone | 0.03 |
| Estradiol | < 0.0012 |
| Danazol | < 0.0012 |
| DHEA | 0.02 |
| DHEA-SO4 | 0.004 |

- Monoclonal and polyclonal anti-estrogen antibodies;
- Specifically for porcin:
- Rabbit anti-LH antibodies;
- Rabbit anti-FSH antibodies;
- Specifically for bovin:
- Rabbit anti-LH antibodies;
- Cobbaye anti-FSH antibodies;
- Specifically for canine:
- Rabbit anti-LH antibodies;
- Rabbit anti-FSH antibodies.

The detection of the analyte Progesterone may be used for ovulation detection, time of insemination, corpus luteum persistence and/or pregnancy detection.

Preferably, the result of a Progesterone test is presented as follows on the calibrated Micro-Scan reader:
a. 1^{st} option: Results displayed in ng/ml for e.g use during clinical trials and for use by veterinarians;
b. 2^{nd} option: For e.g. use by farmer's results is preferably shown in a bar-diagram, e.g. Black Square boxes, as follows:

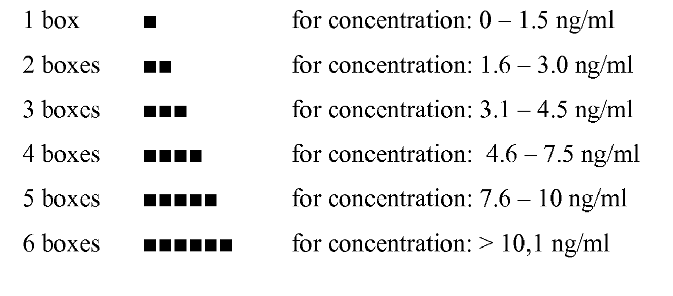

Dependent on the animal the following information can be obtained from the detection of Progesterone in e.g. a blood sample. The concentration of Progesterone can be correlated as follows:
Cows:
   - Estrus cycle: 0.1 - 1 ng/ml;
   - After ovulation: up to 7.0 ng/ml (cycle day 14 to 21);
   - During pregnancy: up to 7 - 8 ng/ml at about 240 days of gestation;
   - Before calving: drop of Progesterone levels starting 2 to 3 weeks before delivery;
Mares:
   - For samples taken at 19-24 or 44-48 (estimated) pregnancy days
   - Non-pregnancy: < 1 ng/ml
   - During pregnancy: > 1.5 ng/ml and much higher
Sows:
   - Follicular phase: < 1 ng/ml
   - Luteal phase: 5 up to 35 ng/ml
Bitches:
   - Estrus cycle: > 2 ng/ml
   - Ovulation: up to 5 ng/ml
   - Ovulation can occur on Day 7 or even at DAY 27 of the estrus cycle. Therefore daily monitoring is important.
   - For ovulation purposes and time of insemination: the only interest is Progesterone 5 to 10 ng/ml.

LH surge, in e.g. blood samples, may be used for predicting time of ovulation. Dependent on the animal the concentration of LH can be correlated as follows:
Cows:
   - LH baseline: 0.5 - 5 ng/ml
   - LH surge: 10 - >15 ng/ml
Mares:
   - For samples taken at 19-24 or 44-48 (estimated) pregnancy days
   - Non-pregnancy: < 1 ng/ml
   - During pregnancy: > 1.5 ng/ml and much higher Sows:
   - Pro-estrus: 0.2 up to 2 ng/ml
   - LH Surge: > 9 ng/ml
Bitches:
   - Pro-estrus 1.4 +/- 0.1 ng/ml
   - LH surge 7.5 +/- 0.8 ng/ml

Detection of FSH, in e.g. blood samples, may be used for follicular reserve on the ovaries or detection of cysts (in combination with LH)

Estradiol is a steroid hormone which peaks about 48 hours before ovulation and is very useful to detect for measuring follicular maturity. Detection of Estradiol may be used for reflecting follicular maturity.
Cows:
   - Basal levels 10 up 80 pg/ml
Mares:
   - For samples at more than 80 days of (estimated) pregnancy
   - Non-pregnancy
   - During pregnancy: 350 up to 800 pg/ml
Bitches:
   - Pro-estrus: 10 up 40 pg/ml
   - Pre-ovulatory peak: 45 up to 115 pg/ml

Prolactin is a lactation hormone, which plays an important role in infertility and in early pregnancy (placental artery formation).
Cows:
   - A wide range in ng/ml of Prolactin levels on cows can be observed, depending if the calf is suckling or not (from 30 up to > 200 ng/ml).

SHBG is a marker of infertility.

Testosterone is a marker of fertility in males and an infertility marker in females.

### ABBREVIATIONS

Artificial Insemination (AI)
Luteinizing Hormone (LH)
Follicle Stimulating Hormone (FSH)
Corpus Luteum (CL)
Sex Hormone Binding Globulin (SHBG)
Prolactin (PRL)

### REFERENCES

Senger; 1994; The estrus detection problem: new concepts, technologies, and possibilities. J Dairy Sci, 1994:77:3185
Trimberger: Breeding efficiency in dairy cattle from artificial insemination at various intervals before and after ovulation. 1948; Univ Nebraska Ag. Exp. Sta. Res. Bull. 153:3
Walker et al.: Time of ovulation relative to mounting activity in the dairy cattle. 1996 J. Dairy Sci.:79:155
Brackett et al. Fertilization and early development of cow ova. 1980: Biol. Reprod.:23:189
W. Leidl et al, Theriogeology 1976
E.H. Gils et al., J Immunology Methods 2002

## Claims

1. A lateral flow immunoassay device for predicting optimum insemination time and/or pregnancy detection of an animal by determining in a body fluid sample of said animal the presence and/or concentration of at least one analyte of interest selected from the group comprising progesterone, lutenizing hormone LH, follicle stimulating hormone FSH, prolactin PRL, sex hormone binding globuline SHBG, estradiol and/or testosterone, said device comprising
- a sample application pad for applying said body fluid sample,
- a sample filtering membrane for separating a liquid fraction from the body fluid sample, said liquid fraction containing an analyte of interest,
- a conjugate release pad containing labelled detection molecules that specifically bind the analyte of interest to form analyte-conjugate complexes,
- an analytical membrane containing a test line with immobilized analyte-specific molecules for immobilizing analyte-conjugate complexes and a control line with immobilized conjugate-specific molecules for immobilizing non-bound conjugate,
wherein said analyte-specific molecules comprise at least one antibody selected from the group of anti-progesterone, anti-lutenizing hormone LH, anti-follicle stimulating hormone FSH, anti-prolactin PRL, anti-sex hormone binding globuline SHBG, anti-estradiol and/or anti-testosterone antibodies.

2. Method for predicting optimum insemination time of an animal, by determining in a body fluid sample of said animal the presence and/or concentration of at least one analyte of interest selected from the group comprising progesterone, lutenizing hormone LH, follicle stimulating hormone FSH, prolactin PRL, sex hormone binding globuline SHBG, estradiol and/or testosterone, the method comprising a lateral flow immunoassay.

3. Method according to claim 2, whereby performing said lateral flow immunoassay comprises
- applying said body fluid sample to a sample application pad,
- separating a liquid fraction containing said analyte from said body fluid sample on a sample filtering membrane,
- allowing said analyte to flow through a conjugate release pad and to interact with said conjugate and form analyte-conjugate complexes,
- allowing further said analyte to flow through an analytical membrane containing a test line with analyte-specific molecules for immobilizing analyte-conjugate complexes and a control line for immobilizing non-bound conjugate.

4. Method according to claim 2 or 3, whereby at least one antibody selected from the group of anti-progesterone, anti-lutenizing hormone LH, anti-follicle stimulating hormone FSH, anti-prolactin PRL, anti-sex hormone binding globuline SHBG, anti-estradiol and/or anti-testosterone antibodies is used in said immunoassay as analyte-specific molecule.

5. Method according to any of claims 2 to 4, whereby said body fluid sample is a blood sample taken from the animal.

6. Method according to any of claims 2 to 5, whereby said body fluid sample consist of a drop of blood directly applied to say lateral flow immunoassay.

7. Method according to any of claims 2 to 6, whereby at least one analyte-specific monoclonal antibody is used in said immunoassay.

8. Method according to any of claims 2 to 7, whereby presence and/or concentration of analyte in the body fluid sample is determined by determining the colour intensity of the test line.

9. Method according to any of claims 2 to 8, whereby presence and/or concentration of progesterone in a blood sample taken from an animal is determined using lateral flow immunoassay with a anti progesterone specific monoclonal antibody.

10. Diagnostic kit for determining the presence and/or the concentration of at least one analyte of interest selected from the group of progesterone, lutenizing hormone LH, follicle stimulating hormone FSH, prolactin PRL, SHBG, estradiol and/or testosterone in a body fluid sample of an animal comprising a lateral flow immunoassay device of claim 1.

11. Use of an antibody selected from the group of anti-progesterone, anti-lutenizing hormone LH, anti-follicle stimulating hormone FSH, anti-prolactin PRL, anti-sex hormone binding globuline SHBG, anti-estradiol and/or anti-testosterone antibodies in a test for pregnancy detection, predicting timing of ovulation of an animal, period of fertility of an animal and/or optimum insemination time of an animal.
